# EUROPÄISCHE PATENTANMELDUNG

(11) **EP 2 272 387 A1**
(43) Veröffentlichungstag der Anmeldung: **12.01.2011**
(21) Anmeldenummer: 10006992.1
(22) Anmeldetag: 07.07.2010
(51) Int. Cl.: A24F 1/16

(54) **Pfeifenaufsatz**

(30) Priorität: 09.07.2009 AT 10812009
(71) Anmelder: Werner, Daniel Sherlock, 4400 Steyr (AT)
(72) Erfinder: Werner, Daniel Sherlock, 4400 Steyr (AT)

(57) **Zusammenfassung**

Die Erfindung betrifft einen Pfeifenaufsatz mit externer Beheizung zur Erzeugung von dampfförmigem, aroma- und/oder wirkstoffhaltigem Aerosol aus Pflanzenmaterial, bestehend aus einem beidseitig offenen und durchgehend hohlen Längsstück (1), das an einem Ende eine weitestgehend luftdichte Verbindung (2) zum Anbringen an einem Pfeifenkopf (3) aufweist, ein Pfeifenaufsatz der die vollkommen homogene Durchlüftung und Erhitzung des Pflanzenmaterials und eine maximale Wirkstoffausbeute garantieren soll, wobei oberhalb der Verbindung (2) eine düsenartige Verengung des Innendurchmessers (4) vorgesehen ist und wobei der Pfeifenaufsatz auch eine weitere Verengung des Innendurchmessers (6) oberhalb der düsenartigen Verengung (4) aufweisen kann. Durch Platzieren einer Hitzequelle knapp oberhalb des Endes (5) oder durch seitliches Erhitzen des Längsstückes (1) und gleichzeitiges Inhalieren durch den Pfeifenkopf (3) wird die durch das Ende (5) in den Aufsatz eindringende Luft erhitzt und vor dem Auftreffen auf das Pflanzenmaterial durch die düsenartige Verengung (4) in Turbulenzen versetzt, wodurch sich der Heißluftstrom gleichmäßig im gesamten Pfeifenkopf verteilt und das Pflanzenmaterial zusätzlich aufwirbelt.

## Beschreibung

Die Erfindung betrifft einen Pfeifenaufsatz mit externer Beheizung zur Erzeugung von dampfförmigem, aroma- und/oder wirkstoffhaltigem Aerosol aus Pflanzenmaterial, bestehend aus einem beidseitig offenen und durchgehend hohlen Längsstück, das an einem Ende eine weitestgehend luftdichte Verbindung zum Anbringen an einem Pfeifenkopf aufweist. Der Begriff "Aerosol" steht für die feinste Verteilung fester oder flüssiger Stoffe in Gas, zum Beispiel Rauch, Nebel oder Dampf. Aroma- und/oder Wirkstoffdampftherapien können bei unterschiedlichsten Erkrankungen (z.B. der Atemwege) sowie bei der Rauchentwöhnung zur Anwendung kommen.

Das Verdampfen der Aroma- und/oder Wirkstoffe einer Pflanze findet bei Temperaturen unterhalb des Brennpunktes statt und die Inhalation des dampfförmigen Aerosols stellt eine gesündere Alternative zum Rauchen einer Pflanze dar. Nachdem es nicht zur Verbrennung des Pflanzenmaterials kommt, gelangen bei der Inhalation der Dämpfe auch keine ungewollten und gesundheitsschädigenden Verbrennungsnebenprodukte in die Lungen. Das einfachste Verfahren zur Erzeugung von dampfförmigem Aerosol aus Pflanzenmaterial stellt dessen Erhitzung über Konduktion bei stehender Luft dar. Bei Erreichen der notwendigen Temperatur beginnt das Pflanzenmaterial Dämpfe auszusondern, die in weiterer Folge eingeatmet werden können. Ein effizienteres Verfahren, sowohl was die Wirkstoffausbeute als auch die Qualität des dampfförmigen Aerosols betrifft, stellt der gezielte Einsatz eines Heißluftstromes dar, welcher am Pflanzenmaterial vorbeigeführt wird und es über Konvektion erhitzt. Dies ist auch bei der vorliegenden Erfindung der Fall.

Es existieren verschiedene Geräte, die Aroma- und/oder Wirkstoffdämpfe (dampfförmiges, aroma- und/oder wirkstoffhaltiges Aerosol) durch einen am Pflanzenmaterial vorbeigeführten Heißluftstrom erzeugen. Als ausgereifteste Lösung für ein auf dem Heifiluftstromprinzip basierendes Gerät gilt zur Zeit eine vulkanförmige Anwendung mit der Patentschrift DE 198 03 376 C1 (EP0933093A2, US 6,513,524 B1). Wie auch bei der genannten Anmeldung der Fall, wird eine Vielzahl der auf dem Heißluftstromprinzip beruhenden Geräte elektrisch betrieben. Sie eignen sich vor allem für den häuslichen Gebrauch, einerseits weil man für den Betrieb eine geeignete Stromquelle benötigt und andererseits wegen ihrer Größe.

Die vorliegende Erfindung reiht sich in eine Lösungsansatz ein, wo das Problem der Energieversorgung über den Gebrauch einer externen Hitzequelle gelöst wird, welche in den meisten Fällen eine Flamme ist. Die Verwendung eines herkömmlichen Feuerzeugs als Hitzequelle ermöglicht das unproblematische Mitnehmen des Hitzespenders. Ebenso wie bei der vorliegenden Erfindung werden die meisten der bekannten Ausführungen von Geräten mit externer Hitzequelle zur Erzeugung von dampfförmigem Aerosol aus Pflanzenmaterial auf ein handliches Taschenformat reduziert, um in Verbindung mit einem herkömmlichen Feuerzeug eine tragbare und günstige Alternative zu größeren und elektrisch betriebenen Geräten darzustellen. Eine Ausnahme stellt diesbezüglich das in der Anmeldung US6354301 beschriebene zweiteilige Gerät dar (vertrieben unter dem Namen 'Vriptech'), das für den Gebrauch mit einer Heißluftpistole vorgesehen ist. Dies erschwert aufgrund der Größe einer Heißluftpistole das unkomplizierte Mitnehmen, macht eine Stromquelle notwendig und ist auch aus gesundheitlicher Sicht bedenklich, da die für die Inhalation bestimmte Luft im Inneren einer herkömmlichen Heißluftpistole an toxischen Bestandteilen vorbeiströmt.

Zu den bekannten flammenbetriebenen Dampfinhalationsapparaten zählen etwa die in der Patenschrift US7415982 beschriebene Pfeife (vertrieben unter dem Namen 'Ubie') und die in den Patentschriften US7434584 und US 2009/0032034 A1 beschriebene Pfeife (vertrieben unter dem Namen 'Vaporgenie').

Es handelt sich bei allen bekannten Modellen um beidseitig offene und hohle Längsstücke, die so konstruiert sind, dass ein in das Längsstück eindringender Luftstrom mit einer externen Hitzequelle auf die geeignete Temperatur für die Verdampfung der Aroma- und/oder Wirkstoffe des gewählten Pflanzenmaterials erhitzt werden kann. Dem Anmeldungsgegenstand am nächsten kommt sicher die in den Patentschriften US7434584 und US 2009/0032034 A1 beschriebene Pfeife, da die darin beschriebene Filtereinheit dezidiert zur Verwendung als eigenständiger Pfeifenaufsatz konzipiert ist und im Dokument US 2009/00320034 auch unterschiedliche Verbindungen zum Anbringen des Aufsatzes auf diversen Pfeifenköpfen angeführt sind.

Ein zentrales Problem der bekannten flammenbetriebenen Dampfinhalationsapparate ist, dass die erhitzte Luft nicht vollkommen gleichmäßig durch das Pflanzenmaterial strömt und daher eine unregelmäßige Verdampfung der Wirkstoffe stattfindet. Am stärksten wird bei den bekannten Modellen die oberste Schicht des im Pfeifenkopf platzierten Pfianzenmateriais erhitzt, also jene Schicht, auf die der Heißluftstrom zuerst trifft. Je weiter der Heißluftstrom durch das Pflanzenmaterial zieht, desto stärker wird er abgekühlt. Bei den seitlich am Pfeifenkopf anllegenden Pflanzenresten trifft man bei den bekannten Modellen auf dasselbe Problem. Der Heißluftstrom durchströmt das Zentrum des Pfeifenkopfs, erreicht aber den äußersten Rand entweder gar nicht oder nicht in derselben Stärke wie das in der Mitte der Fall ist. Diese Probleme sind allgemein bekannt. Der im Dokument US2009/0032034 beschrieben Anmeldungsgegenstand bietet diesbezüglich zumindest eine Lösung für das im Pfeifenkopf seitlich anliegende Pflanzenmaterial an. Dabei handelt es sich um eine Scheibe mit Löchern (Bezugszeichen 70 in Fig. 11, [0072] - [0073]), welche der Filtereinheit in der genannten Anmeldung vorangestellt wird. Das Problem der geringeren Erhitzung der unteren Schichten des Pflanzenmaterials ist bislang aber bei keiner flammenbetriebenen Pfeife zur Erzeugung von dampfförmigem Aerosol gelöst worden.

Die bekannten Modelle weisen keine Vorrichtung auf, die garantiert, dass sowohl das seitlich am Pfeifenkopf anliegende als auch das im Pfeifenkopf zuunterst liegende Pflanxenmaterial genauso stark erhitzt werden und genauso stark dem eindringenden Luftstrom ausgesetzt werden wie es beim Pflanzenmaterial im Zentrum und im oberen Teil des Pfeifenkopfes der Fall ist. Ungleichmäßig verdampftes Pflanzenmaterial wirkt sich aber nachteilig aus auf die Qualität des Aerosols und auf die erzielte Wirkstoffausbeute.

Der vorliegenden Erfindung liegt die Aufgabe zugrunde, einen Pfeifenaufsatz mit externer Beheizung zur Erzeugung von dampfförmigem, aroma- und/oder wirkstoffhaltigem Aerosol aus Pflanzenmaterial der eingangs geschilderten Art so weiterzubilden, dass er die vorgenannten Nachteile möglichst überwindet, und eine in allen Teilen und Schichten des Pfeifenkopfs vollkommen gleichmäßige und homogene Verdampfung der Wirkstoffe des Pflanzenmaterials garantiert.

Die Aufgabe wurde durch eine düsenartige Verengung des Innendurchmessers oberhalb der Verbindung des Aufsatzes mit dem Pfeifenkopf gelöst.

Durch die düsenartige Verengung wird erreicht, dass der zuvor erhitzte Luftstrom beim Passieren der Düse in Turbulenzen versetzt wird, die dann auf das Pflanzenmaterial treffen und eine homogene Erhitzung des Pflanzenmaterials bewirken. Der gesamte Füllraum für das Pflanzenmaterial wird gleichmäßig vom Heißluftstrom erfasst und zusätzlich wird das im Normalfall getrocknete und zerkleinerte Pflanzenmaterial aufgewirbelt. Die düsenartige Verengung sorgt also in doppelter Hinsicht für einen homogenen und vollständigen Verdampfungsprozess: Erstens wird heiße, in den Pfeifenkopf eindringende Luft in Turbulenzen versetzt und gestreut und zweitens wird im Pfeifenkopf das Pflanzenmaterial selbst bewegt. Das Problem des an der Wand des Pfeifenkopfs anliegenden und nur unzureichend vom Heißluftstrom erreichten Pflanzenmaterials ist ebenso beseitigt wie jenes der unteren Schichten des Pflanzenmaterials, die bei den bekannten Modellen nicht in gleicher Weise dem Heißluftstrom ausgesetzt sind wie die oberen Schichten. Die düsenartige Verengung und die durch sie hervorgerufenen Turbulenzen des Heißluftstroms sorgen beim Inhalieren für eine konstante Bewegung des Pflanzenmaterials im Pfeifenkopf, beheben das Problem unzureichend erfasster äußerer und/oder unterer Schichten und gewährleisten homoge Temperatur- und Durchlüftungsverhältnisse im Pfeifenkopf.

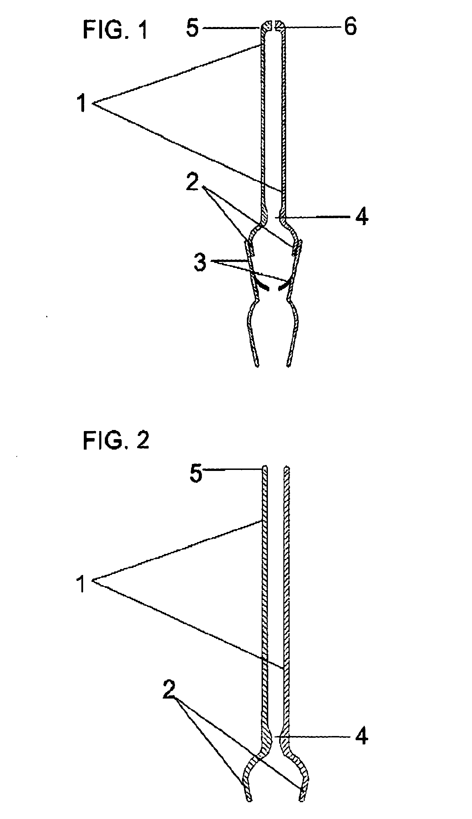

Es sind zwar auch bei anderen bekannten Anmeldungen Verengungen des Innendurchmessers des Längsstückes vorgesehen, aber sie erfüllen immer einen gänzlich anderen Zweck. So etwa die im Dokument US 2009/0032034 beschriebe Verengung (Bezugszeichen 106 in Fig. 24, [0081]), wo es sich bei der Verengung um einen Vorsprung im Filtergehäuse handelt, um eine Filtereinheit zu befestigen. Auch die in der Patentschrift US 6148826 beschriebene Verengung (Bezugszeichen Nr 24 in Fig. 1, 2. und 3., [Seite 2, Zeile 15 - 20] dient lediglich der Aufteilung des beschriebenen Längsstückes in eine hintere Rauchkammer und eine vordere Füllkammer für das Pflanzenmaterial. Die Verengung hält dabei das Pflanzenmaterial an seinem Platz. Dazu kommt, dass der Benutzer beim zitierten Anmeldungsgegenstand am Ende der hinteren Rauchkammer anzieht, was in klarem Gegensatz zu dem hier beschriebenen Pfeifenaufsatz bedeutet, dass die Verengung erst dann zum Einsatz kommt, wenn die Luft schon am Pflanzenmaterial vorbei ist. In der Patentschrift US 7415982 ist ebenfalls eine Verengung des Innendurchmessers des Längsstückes vorgesehen (Bezugszeichen 30 in Fig. 1 und Fig. 2), mit der einzigen und - wie auch im Abstract und im ersten Anspruch betont - ausschließlichen Funktion, als physische Barriere für das Pflanzenmaterial zu dienen und es im Inneren des Längsstücks zu stabilisieren.
Die bekannten Verengungen sind also hinsichtlich ihrer Funktion in keiner Weise vergleichbar mit der hier beschriebenen düsenartigen Verengung. Die hier beschriebene düsenartige Verengung dient nicht als Befestigung, Trennung, Teilung o. Ä., sondern löst durch gezieltes Einsetzen der durch sie verursachten Turbulenzen des Heißluftstromes das bisher ungelöste Problem der unvollständigeren Verdampfung äußerer und unterer Bereiche des im Pfeifenkopf platzierten Pflanzenmaterials.

Der Pfeifenaufsatz ist zudem als einziger seiner Art sowohl für das direkte als auch das indirekte Erhitzen des Luftstroms konzipiert. Das direkte Erhitzen wie etwa bei den aus den Dokumenten US7415982, US7434584 und US 2009/0032034 A1 bekannten Modellen bringt den Heißluftstrom schneller auf die gewünschte Temperatur, aber zugleich werden beim Einsatz einer Flamme potentiell schädliche Verbrennungsrückstände der Flamme mit eingeatmet. Das indirekte Erhitzen des Luftstroms dauert etwas länger, schließt aber Verunreinigungen des Heißluftstroms durch Verbrennungsrückstände der Flamme aus. Beispiele für indirekt erhitzte Luftströme von Vaporisiergeräten der hier behandelten Art sind etwa der in der Patentschrift EP1779886 (AT502169) beschriebene Tascheninhalator (vertrieben unter dem Namen 'Vapbong'), ein unter dem Namen 'Vapman' vertriebenes Produkt und ein unter dem Namen 'lolite' vertriebenes Produkt.

Wenn beim Anmeldegegenstand direktes Erhitzen des Luftstroms bevorzugt wird, dann muss die Hitzequelle knapp über das dem Verbindungsende entgegengesetzte Ende gehalten werden. Beim Saugen an der Pfeife strömt die Luft vor dem Eindringen in den Pfeifenaufsatz an der Hitzequelle vorbei und wird direkt erhitzt, danach setzt sie ihren Weg ins Innere des Aufsatzes fort. Wenn indirektes Erhitzen des Luftstroms bevorzugt wird, dann wird die Hitzequelle seitlich auf das beidseitig offene und hohle Längsstück gerichtet. Beim Saugen an der Pfeife wird die Luft nach dem Eindringen in den Aufsatz durch das heiße Längsstück indirekt erhitzt, ohne direkt mit der externen Hitzequelle in Berührung zu kommen. Das Längsstück dient beim direkten Gebrauch der Hitzequelle als Abstandhalter zum Pflanzenmaterial, um den direkten Kontakt zwischen beispielsweise einer Flamme und dem Pflanzenmaterial zu vermeiden. Beim indirekten Gebrauch der Hitzequelle sorgt es dafür, dass ausreichend Angriffsfläche für das seitliche Erhitzen vorhanden ist um den ungewollten direkten Kontakt zwischen Luftstrom und Hitzequelle zu vermeiden. Die Länge kann beliebig gewählt werden, wobei neben der Funktion als Abstandhalter zwischen Flamme und Pflanzenmaterial und der Funktion, als ausreichende Angriffsfläche für die externe Hitzequelle zu dienen, die Länge auch hinsichtlich der Wärmespeicherkapazität für die effiziente Erhitzung des Luftstroms von Bedeutung ist.

Die düsenartige Verengung des Innendurchmessers Ist sowohl für den direkten als auch den indirekten Gebrauch der Hitzequelle von entscheidender Bedeutung, da sie immer für eine gleichmäßige Verteilung des Heißluftstroms und das Aufwirbeln des Pflanzenmaterials sorgt.

Als Material für den Pfeifenaufsatz kommt vorzugsweise hitzeresistentes Glas in Frage, grundsätzlich ist aber auch die Verwendung einer Reihe von anderen hitzebeständigen Materialen wie etwa Keramik oder Metall vorstellbar Die Verbindung zum Anbringen des Pfeifenaufsatzes an einem Pfeifenkopf bei der Verwendung von Glas ist vorzugsweise ein Normschliffkern. In den vergangenen Jahren sind Glaspfeifen immer populärer geworden, und unabhängig vom Hersteller oder von der Marke optieren zahlreiche Modelle bei der Wahl des Pfeifenkopfes für ein Glasrohr mit innerem Kontaktverbindungsschliff, eine sogenannte Schliffhülse. Diese Teile werden in Standardgrößen industriell gefertigt, haben den Vorteil eines leicht zu reinigenden glatt geschliffenen Innenraums und bieten sich aufgrund ihrer präzisen zumeist konischen Ausführung und ihrer geringen Kosten für die Verwendung als Pfeifenkopf regelrecht an. Normalerweise werden diese Schliffhülsen ('Weibchen') zusammen mit Schliffkernen ('Männchen') als luftdichte Steckverbindungen zwischen Glasröhre eingesetzt, das System funktioniert aber auch mit anderen Materialien. Die Schliffkerne sind dementsprechend Röhren mit einem äußeren Verbindungsschliff der genau in den Verbindungsschliff der Schliffhülse passt. Die Verwendung einer Schliffhülse als Pfeifenkopf stellt eine Zweckentfremdung dar Zugleich erlaubt dieser Umstand aber das unkomplizierte und luftdichte Anbringen von allen möglichen Geräten am Pfeifenkopf, solange sie einen passenden äußeren Verbindungsschtiff aufweisen.

Art und Ausführung der Verbindung sind aber in keiner Weise beschränkt und für das einwandfreie Funktionieren des Anmeldegegenstandes nicht von entscheidender Bedeutung, Steckverbindungen sind ebenso vorstellbar wie Schraubverbindungen oder O-Ringverbindungen oder Einrastverbindungen. Es muss lediglich gewährleistet sein, dass die Verbindung weitestgehend luftdicht ist und beim Ziehen an der Pfeife die Luft zumindest teilweise durch den Aufsatz strömt und der Aufsatz nicht etwa durch undichte Stellen der Verbindung überbrückt bzw. außer Funktion gesetzt wird. Kleinere undichte Stellen der Verbindung können in Kauf genommen und gegebenenfalls sogar bewusst zur Abkühlung des Heißluftstroms eingesetzt werden. Im Dokument US 2009/0032034 wird ein Vielzahl von möglichen Verbindungsarten für den dort beschriebenen Anmeldungsgegenstand beschrieben welche ohne Ausnahme auch für den hier beschriebenen Pfeifenaufsatz Anwendung finden können. Der Pfeifenaufsatz ist universell einsetzbar und nicht an ein bestimmtes Pfeifenmodell gebunden.

Des weiteren ist erfindungsgemäß eine weitere Verengung des Innendurchmessers oberhalb der düsenartigen Verengung vorgesehen.

Eine weitere Verengung des Innendurchmessers oberhalb der ersten düsenartigen Verengung kann das Entweichen heißer Luft durch das dem Verbindungsschliff entgegengesetzte Ende des beidseitig offenen und durchgehend hohlen Längsstückes reduzieren. Dies ist vor allem beim seitlichen Erhitzen des Aufsatzes wichtig, also beim Indirekten Einsatz der externen Hitzequelle. Es bildet sich ein Stauraum für die erhitzte Luft zwischen der düsenartigen Verengung des Innendurchmessers oberhalb des Verbindufigsschliffes und der weiteren Verengung des Innendurchmessers des beidseitig offenen und durchgehend hohlen Längsstückes.

Zudem ermöglicht eine derartige weitere Verengung des Innendurchmessers die Steuerung des Durchzugs. Je kleiner die düsenartige Verengung ist, desto größer ist auch die Bremswirkung für den eindringenden Luftstrom und desto mehr Widerstand fühlt der Benutzer beim Inhalieren durch die Pfeife. Anders ausgedrückt, je kleiner die Verengung desto heftiger muss der Benutzer anziehen, um das selbe Luftvolumen einzuatmen, das er bei einem größeren Durchmesser durch leichteres Anziehen einatmet. Nun kann es aber von großem Interesse sein, den Durchzug zu erschweren und dadurch zu verhindern, dass der Benutzer zu große Mengen an heißer Luft einatmen und eventuell dadurch zu Schaden kommt kann. Theoretisch kann auch die erste düsenartige Verengung oberhalb der Verbindung zum Pfeifenkopf die Aufgabe der Durchzugsreduzierung erfüllen. Die Kombination mit einer weiteren Verengung ermöglicht aber eine exaktere Feinabstimmung. So kann es zum Beispiel sein, dass für die erste düsenartigen Verengung des Innendurchmessers oberhalb des Verbindungsschliffes ein zur Erzeugung der gewünschten Turbulenzen idealer Durchmesser gewählt wird, der aber den Durchzugs nicht wunschgemäß reduziert. Dies kann durch einen geringeren Durchmesser der weiteren Verengung des Innendurchmessers des beidseitig offenen und durchgehend hohlen Längsstückes ausgeglichen werden, der in diesem Fall die Funktion der Durchzugsreduzierung zukommt. Diese weitere Verengung kann sich an einem beliebigen Punkt des Längsstückes oder direkt an dem der Verbindung entgegengesetzten Ende befinden.

Weiters ist erfindungsgemäß vorgesehen, dass das hohle Längsstück oberhalb der Verbindung gewunden ist.

Ein gewundenes Längsstück oberhalb der Verbindung bietet die Möglichkeit, die zu erhitzenden Gesamtfläche und die Wärmespeicherkapazität zu vergrößern, ohne den Abstand zwischen den zwei Enden des beidseitig offenen und durchgehend hohlen Längsstückes vergrößern zu müssen.

Erfindungsgemäß ist zudem im Inneren des Aufsatzes zwischen der düsenartigen Verengung und dem Ende des Längsstückes im Bereich der Verbindung ein abnehmbares, direkt am Aufsatz fixiertes Gitter vorgesehen, welches einen Füllraum für Pflanzenmaterial im Inneren des Aufsatzes schafft und einen eigenen Pfeifenkopf überflüssig macht. Es besteht die Möglichkeit, das Gitter aus dem Aufsatz herauszunehmen, das Pflanzenmaterial direkt im Inneren des Aufsatzes im Bereich des Verbindungsschliffes zu platzieren und das Gitter wieder einzusetzen. Dadurch entfällt die Notwendigkeit eines eigenen Pfeifenkopfes, und der Pfeifenaufsatz kann somit direkt in ein beliebiges Rohr oder beispielsweise in einen Silikonschlauch gesteckt werden und ist unter Einsparung der Kosten für einen Pfeifenkopf voll einsatzfähi g. Zusätzlich sorgt das vorzugsweise aus Metall gefertigte Gitter für eine homogene Temperaturverteilung. Der Anmeldegegenstand des Dokuments US 2009/0032034 sieht zwar ebenfalls ein Gitter für die Platzierung und Stabilisierung des Pflanzenmaterials im Pfeifenkopf vor (Bezugszeichen 30 in Fig. 1, Fig. 2, Fg. 4a/4b bis Fig. 7, Fig. 21, Fig. 22, Fig. 26), dieses Gitter wird aber ebenso wie bei den anderen bekannten Modellen immer im Bereich des Pfeifenkopfes und am Pfeifenkopf fixiert. Das Anbringen des Gitters direkt am Aufsatz und die Schaffung eines eigenen Füllraums im Inneren des Aufsatzes ist demgegenüber ein klarer Vorteil.

Zudem ist erfindungsgemäß vorgesehen, dass das beidseitig offene und durchgehend hohle Längsstück eine Markierung des zu erhitzenden Bereichs aufweist.

Die Markierung des zu erhitzenden Bereichs ermöglicht den Benutzerinnen einerseits das Lokalisieren des idealen Heizbereiches und trägt andererseits dazu bei, Verbrennungen durch Berührung des noch nicht abgekühlten Pfeifenaufsatzes zu vermeiden.

### Figurenübersicht

Fig.1 zeigt einen Längsschnitt durch einen mögliche Ausführungsform des an einem Pfeifenkopf angebrachten Pfeifenaufsatzes
Fig. 2 zeigt einen Längsschnitt durch eine mögliche Ausführungsform des Pfeifenaufsatzes
Fig. 3 zeigt einen Längsschnitt durch eine weitere mögliche Ausführungsform des Pfeifenaufsatzes

In dem dargestellten Ausführungsbeispiel des Pfeifenaufsatzes in Fig. 1 ist das beidseitig offene und durchgehend hohle Längsstück 1 zu sehen, des weiteren die Verbindung 2 mit dem Pfeifenkopf 3, die düsenartige Verengung 4 des Innendurchmessers oberhalb der Verbindung 2, und eine weitere Verengung des Innendurchmessers 6 oberhalb der düsenartigen Verengung 4. Die Verengung 6 befindet sich in diesem Fall an dem der Verbindung entgegengesetzten Ende 5 des durchgehend hohlen Längsstückes 1. Der Verbindungstyp im gewählten Ausführungsbeispiel entspricht einer Steckverbindung zwischen einer konischen Normschliffhülse und einem ebenfalls konischen Normschliffkem. Solange die Verbindung weitestgehend luftdicht ist, ist ihre konkrete Ausführung für das korrekte Funktionieren des Aufsatzes nicht von Bedeutung und daher auch nicht erfindungsrelevant. Es muss lediglich gewährleistet sein, dass beim Ziehen an der Pfeife die Luft zumindest teilweise durch den Aufsatz strömt und der Aufsatz nicht vollkommen überbrückt bzw. außer Kraft gesetzt wird. Die düsenartige Verengung 4 des Innendurchmessers oberhalb der Verbindung 2 kann in Form und Länge ebenfalls variieren. Im Fall des optionalen Pfeifenkopfes 3 aus dem Ausführungsbeispiel wird das Pflanxenmaterial zuerst in die Schliffhülse gefüllt, wo es auf dem Boden des Pfeifenkopfes zu liegen kommt. Der Boden des Pfeifenkopfes 3 weist im Zentrum einen Öffnung für den Durchzug des Luftstroms auf. Danach wird der Pfeifenaufsatz aufgesteckt, wodurch das Pflanzenmaterial im Pfeifenkopf 3 zwischen dem Boden des Pfeifenaufsatzes und der düsenartigen Verengung 4 des Innendurchmessers oberhalb der Verbindung 2 zu liegen kommt.

Die in Fig. 2 dargestellte mögliche Ausführungsform beinhaltet die Basisversion des Pfeifenaufsatzes mit den für sein korrektes Funktionieren unerlässlichen Bestandteilen: das beidseitig offene und durchgehend hohle Längsstück 1, die weitestgehend luftdichte Verbindung 2 und die düsenartige Verengung des Innendurchmessers 4 oberhalb der Verbindung 2. Der Pfeifenaufsatz wird vorzugsweise aus hitzeresistentem Glas hergestellt, kann aber auch aus anderen hitzeresistenten Materialien wie zum Beispiel Keramik oder Metall angefertigt werden. Die Verbindung 2 ist in der gewählten Darstellung ein konischer Normschliffkern der zum Anbringen an einem Pfeifenkopf in Form einer konischen Normschliffhülse geeignet ist. Die düsenartige Verengung kann sich an einem beliebigen Ort oberhalb der Verbindung 2 des beidseitig offenen und durchgehend hohlen Längsstückes 1 befinden, so auch an seinem Ende 5.

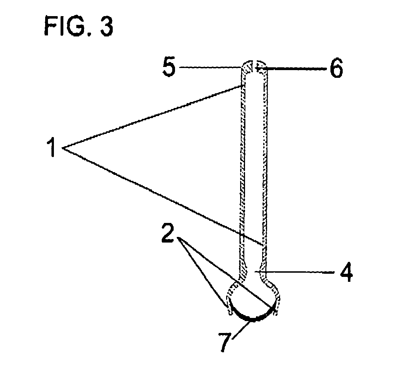

Fig. 3 enthält enthält eine möglichen Ausführungsform des Pfeifenaufsatzes in Anlehnung an Fig. 1 mit einer beispielhaften Darstellung des abnehmbaren Gitters 7 im Bereich der Verbindung 2 im Inneren des beidseitig offenen und durchgehend hohlen Längsstückes 1. Das vorzugsweise aus Metall gefertigte Gitter 2 wird im Ausführungsbeispiel über Druck an der Innenwand des beidseitig offenen und durchgehend hohlen Längsstückes 1 stabilisiert. Das Gitter kann aber auch aus vielen anderen Materialen gefertigt werden. Da der Verdampfungsprozess unterhalb 10 des Brennpunktes des Pftanzenmaterials stattfindet, wären sogar weniger hitzeresistente Materiallen wie etwa Papier vorstellbar. Das abnehmbare Gitter 7 kann herausgenommen werden um das Innere des Pfeifenaufsatzes direkt mit dem Pflanzenmaterial zu füllen. Danach wird das Gitter 7 erneut In den Aufsatz gesteckt und das Gerät ist ebenso bereit für den Betrieb wie es bei der Platzierung des Pflanzenmaterials in einem Pfeifenkopfs der Fall wäre. Der Pfeifenaufsatz wird in diesem Fall in ein geeignetes Rohr oder in einen Silikonschlauch gesteckt, um daran anziehen zu können.

Die Bedienung ist denkbar einfach. Der Pfeifenkopf wird an der Pfeife angebracht wenn er nicht bereits fix mit einer verbunden ist. Theoretisch kann man auch direkt am Pfeifenkopf anziehen, aber eine Verlängerung in Form einer Pfeife oder eines Schlauches erleichtert die Bedienung und sorgt zusätzlich für die Abkühlung des Heißluftstroms vor Eintritt in die Mundhöhle. Egal mit welchem Pfeifenkopf, der Pfeifenaufsatz funktioniert immer auf die selbe Weise. Der Aufsatz sollte so gehalten werden, dass das der Verbindung entgegengesetzte Ende 5 des beidseitig offenen und durchgehend hohlen Längsstückes 1 nach oben zeigt. Diese Ausrichtung des Pfeifenaufsatzes ergibt sich prinzipiell automatisch, da der Aufsatz im Pfeifenkopf steckt und Pfeifenköpfe in der Regel nicht nach unten gehalten werden, da sonst ja der Inhalt des Pfeifenkopfes auf den Boden fallen würde. Wenn die externe Hitzequelle direkt zum Aufheizen des Luftstroms verwendet wird, dann muss sie beim betriebsberelten Gerät direkt über das Ende 5 des beidseitig offenen und durchgehend hohlen Längsstückes 1 gehalten werden. Der Benutzer oder die Benutzerin zieht sodann an der Pfeife bzw. am Pfeifenkopf, wodurch Luft an der Hitzequelle vorbeiströmt und aufgeheizt wird. Durch die düsenartige Verengung des Innendurchmessers 4 oberhalb der Verbindung 2 werden beim so entstandenen Heißluftstrom Turbulenzen erzeugt, die für ein gleichmäßige, hochqualitative und flächendeckende Erwärmung des Pflanzenmaterials sorgen. Die Entstehung von dampfförmigem Aerosol beginnt in Abhängigkeit von der gewählten Pflanze in unterschiedlichen Temperaturbereichen und kann geschmacklich wahrgenommen werden. Der Dampf gelangt über den Pfeifenkopf 3 und den Pfeifenausgang in die Mundhöhle und die Lunge und wird mit zunehmender Erwärmung beim Ausatmen auch immer stärker sichtbar. An diesem Punkt sollte die Hitzequelle vom Pfeifenaufsatz entfernt werden, um das Risiko einer aufgrund der Konstruktion des Pfeifenaufsatzes zwar unwahrscheinlichen aber dennoch möglichen Überhitzung und Verbrennung des Pflanzenmaterials zu vermeiden. Schutz vor diesem Risiko bietet der Pfeifenaufsatz beim direkten Gebrauch der Hitzequelle einerseits durch seine Länge, die den direkten Kontakt der Hitzequelle mit dem Pflanzenmaterial verhindert, und andererseits durch die Kontrolle des Durchzugs durch die Verengungen des Innendurchmessers 2 und 6. Beim indirekten Einsatz der externen Hitzequelle wird diese seitlich auf das beidseitig offene und durchgehend hohle Längsstück 1 gerichtet. Das Längsstück 1 beginnt sich zu erwärmen und der Benutzer oder die Benutzerin wie gehabt an der Pfeife zu ziehen. Das Erreichen der für den Verdampfungsprozess notwendigen Mindesttemperatur wird im Vergleich zum direkten Einsatz der Hitzequelle etwas länger dauern, dafür ist die Inhalation von Verunreinigungen und schädlichen Dämpfen der Hitzequelle aber vollkommen ausgeschlossen. Das Pflanzenmaterial wird in beiden Fällen ausschließlich über Konvektion erhitzt und nur dann konsumiert, wenn an der Pfeife gezogen wird.

## Patentansprüche

1. Pfeifenaufsatz mit externer Beheizung zur Erzeugung von dampfförmigem, aroma- und/oder wirkstoffhaltigem Aerosol aus Pflanzenmaterial mit einem beidseitig offenen und durchgehend hohlen Längsstück (1) das an einem Ende eine weitestgehend luftdichte Verbindung (2) zum Anbringen des Aufsatzes auf einem Pfeifenkopf (3) aufweist, **gekennzeichnet durch** eine düsenartige Verengung (4) des Innendurchmessers oberhalb der Verbindung (2), wodurch die **durch** das entgegengesetzte Ende (5) eindringende und zuvor mit einer externen Hitzequelle erhitzte Luft vor dem Auftreffen auf das Pflanzenmaterial im Pfeifenkopf in Turbulenzen versetzt und eine homogene Durchlüttung und Erhitzung des Pflanzenmaterials sowie eine vollständige Wirkstoffausbeute garantiert wird.

2. Pfeifenaufsatz nach Anspruch 1, **dadurch gekennzeichnet, dass** er eine weitere Verengung des Innendurchmessers (6) oberhalb der düsenartigen Verengung (4) aufweist.

3. Pfeifenaufsatz nach Anspruch 1-2, **dadurch gekennzeichnet, dass** im Inneren des Aufsatzes zwischen der düsenartigen Verengung (4) und dem Ende des Längsstückes (1) im Bereich der Verbindung (2) ein abnehmbares Gitter (7) vorgesehen ist, welches direkt am Pfeifenaufsatz befestigt ist, einen Füllraum für Pflanzenmaterial im Inneren des Aufsatzes schafft und einen eigenen Pfeifenkopf überflüssig macht.

4. Pfeifenaufsatz nach Anspruch 1-3, **dadurch gekennzeichnet, dass** das beidseitig offene und durchgehend hohle Längsstück (1) gewunden ist.

5. Pfeifenaufsatz nach Anspruch 1-4, **dadurch gekennzeichnet, dass** das beidseitig offene und durchgehend hohle Längsstück (1) eine Markierung des zu erhitzenden Bereichs aufweist.
